# EUROPÄISCHE PATENTSCHRIFT

(11) **EP 2 038 062 B1**
(45) Veröffentlichungstag und Bekanntmachung des Hinweises auf die Patenterteilung: **25.05.2016**
(21) Anmeldenummer: 07785576.5
(22) Anmeldetag: 28.06.2007
(51) Int. Cl.: B01L 3/00, B01J 19/00

(54) **MIKROREAKTOREN-ARRAY, VORRICHTUNG MIT EINEM MIKROREAKTOREN-ARRAY UND VERFAHREN ZUR VERWENDUNG EINES MIKROREAKTOREN-ARRAYS**
MICROREACTOR ARRAY, DEVICE COMPRISING A MICROREACTOR ARRAY, AND METHOD FOR USING A MICROREACTOR ARRAY
RÉSEAU DE MICRORÉACTEURS, DISPOSITIF MUNI D'UN RÉSEAU DE MICRORÉACTEURS ET PROCÉDÉ POUR UTILISER UN RÉSEAU DE MICRORÉACTEURS

(30) Priorität: 28.06.2006 DE 102006030068
(43) Veröffentlichungstag der Anmeldung: 25.03.2009
(73) Patentinhaber: M2p-labs GmbH, 52499 Baesweiler (DE)
(72) Erfinder: MÜLLER, Carsten, 52062 Aachen (DE); KLAMMER, Ingo, 52072 Aachen (DE); KENSY, Frank, 52064 Aachen (DE); BÜCHS, Jochen, 52064 Aachen (DE); HOFMANN, Mirko, 52066 Aachen (DE); BUCHENAUER, Andreas, 52072 Aachen (DE); SCHNAKENBERG, Uwe, 52074 Aachen (DE); MOKWA, Wilfried, 47800 Krefeld (DE)
(74) Vertreter: Castell, Klaus
(86) Internationale Anmeldenummer: PCT/DE2007/001143
(87) Internationale Veröffentlichungsnummer: WO 2008/000238

(56) Entgegenhaltungen:
- EP-A- 1 415 710
- WO-A1-2004/016727
- WO-A2-03/103813
- DE-A1- 10 221 565
- DE-A1- 10 238 825
- DE-A1- 10 254 564
- DE-B3- 10 329 820
- DE-C1- 10 041 853
- US-A1- 2003 008 308
- US-A1- 2003 012 657
- US-A1- 2003 064 507
- US-A1- 2004 115 731
- US-A1- 2005 047 967
- US-A1- 2005 129 581
- US-A1- 2005 142 664
- MASTRANGELO C. H., BURNS M.A., BURKE D.T.: "Microfabricated Devices for Genetic Diagnostics", PROCEEDINGS OF THE IEEE, vol. 86, no. 8, 8 August 1998 (1998-08-08) , pages 1769-1787,

## Beschreibung

Die Erfindung betrifft eine Vorrichtung mit einem Mikroreaktoren-Array und ein Verfahren zur Verwendung einer derartigen Vorrichtung. Mittels herkömmlicher Titerplatten lassen sich Mikroreaktoren Arrays von z.B. 6, 24, 48, 96, 384 oder mehr einzelnen Mikroreaktoren realisieren. So wie die Anzahl der Mikroreaktoren stark variiert, kann auch das Volumen der einzelnen Reaktoren unterschiedlich sein. Während schon bei Maßstäben unter 10 ml von Mikroreaktoren gesprochen wird, kann eine weitere Reduzierung des Volumens auf unter 1 ml, unter 500 µl, unter 100 µl oder gar unter 10 µl einige Vorteile mit sich bringen. Das durch die Volumenreduktion erhöhte Oberflächen-Volumen-Verhältnis wird größer und damit ein Sauerstoffeintrag in die Reaktionslösung durch einfache Diffusion einfacher. Die Mikroreaktoren Arrays können bis zur Beendigung der Reaktion kontinuierlich geschüttelt werden. Die individuelle Zudosierung erlaubt die Durchführung unterschiedlicher Versuche in jedem einzelnen Reaktor. Die Erfindung eignet sich insbesondere zur Automatisierung von Screening-Versuchen in einer Fedbatch Betriebsweise, einer kontinuierlichen Betriebsweise und/oder einer pH-Regelung. Sie betrifft im Besonderen mikrobielle, biochemische, enzymatische und chemische Reaktionen. Die Erfindung ermöglicht einen sterilen, aseptischen oder monoseptischen Betrieb.

Als Stellgrößen für die Regelung der Zudosierung können Prozessparameter wie z.B. pH-Wert, gelöster Sauerstoff-, gelöster Kohlendioxid-, Biomassen-, Edukt- und Produktkonzentrationen oder Temperatur verwendet werden. Insbesondere eignet sich im Zusammenhang mit der Erfindung eine nicht-invasive Erfassung der Prozessparameter durch optische oder elektromagnetische Messmethoden durch für den elektromagnetische Strahlung-durchlässigen Boden.

In vielen Bereichen der Biologie, Verfahrenstechnik, Pharmazeutik und Medizin ist ein Screening von biologischen Systemen notwendig (z.B. Selektion von geeigneten biologischen Stämmen, Enzymen oder geeigneten Kulturmedien und -bedingungen). Dabei besteht ein Bedarf an hohen Probendurchsätzen (Parallelisierung der Versuche) und an einer Reduzierung der zum Teil teuren Ausgangsstoffe.

Mit den heute verwendeten Bioreaktoren, wie Schüttelkolben, Kleinfermentern und Reagenzgläsern ist man nicht in der Lage, diesem Bedarf gerecht zu werden. Die etablierten Techniken werden den Anforderungen an die Automatisierung, der Kostenminimierung und dem benötigten hohen Durchsatz nicht gerecht. Gerade bei biokatalytischen Systemen ist der Bedarf an vielen parallelen Versuchen im Mikrolitermaßstab besonders hoch, da solche Prozesse im Allgemeinen langsamer ablaufen und gerade in der Entwicklungsphase teurer als vergleichbare chemische Prozesse sind. Daher besteht die Notwendigkeit, Mikrobioreaktoren zu entwickeln, die auf kleinstem Raum eine geeignete Umgebung für die biologische Kultivierung und biokatalytische Reaktionen liefern. Als wichtige Voraussetzungen für geeignete Betriebsbedingungen sind hierbei zwei Kriterien hervorzuheben: die Möglichkeit die entsprechenden Versuche unter sterilen oder monoseptischen Bedingungen durchzuführen und die Gewährleistung eines für die biologische Kultur oder das biokatalytische Reaktionssystem geeigneten und ausreichenden Stofftransfers (flüssig-flüssig, flüssig-gas, fest-flüssig, fest-gas)

Mikroreaktoren Arrays, wie zum Beispiel Mikrotiterplatten (MTP), bieten eine ideale Plattform, um einen hohen Parallelisierungsgrad zu erreichen. Aufgrund der kleinen Reaktionsvolumina (z.B. <10 µL bis >5 mL pro Kammer), dem hohen Parallelisierungsgrad (z.B. 6 bis 1536 Kammern pro Platte) und der Möglichkeit zur Automatisierung der Kultivierungsprozesse (von Robotern handhabbare Form) stellen Mikroreaktoren Arrays den insgesamt kostengünstigsten und zukunftsträchtigsten Bioreaktor da. Weiterhin ist der Einsatz nicht-invasiver, optischer Messmethoden zur Erfassung von Prozessgrößen in diesem Reaktortyp bereits weit fortgeschritten. Zudem sind die Betriebsbedingungen in geschüttelten MTP hinsichtlich des Stofftransfers (maximale Sauerstofftransferkapazität) bereits gut charakterisiert (Hermann et al., 2002) und somit mit den Betriebsbedingungen in Labor-, Pilot- oder Produktionsreaktoren zu vergleichen.

Gerade aus diesen Gründen kommen in letzter Zeit vermehrt Mikroreaktoren Arrays zum Einsatz. Aktuell werden Mikrotiterplatten bereits zum Screening von biologischen Systemen eingesetzt. Dazu werden die einzelnen Reaktionskammern befüllt, angeimpft und auf einem Rotationsschüttler inkubiert. Durch die Schüttelbewegung wird der Eintrag von Sauerstoff in die Reaktionsflüssigkeiten verbessert und eine Durchmischung der Reaktionsflüssigkeiten erreicht. Neben der Schüttelbewegung gibt es auch andere Möglichkeiten der Flüssigkeitsdurchmischung in Mikrotiterplatten welche in Frage kommen (z.B. die Flüssigkeitsbewegung durch Schallwellen). Um das System steril zu halten werden die Mikrotiterplatten durch eine luftpermeable Membran (Porengröße < 0,2 µm) oder luftdichte Folie oder eine Deckelkonstruktion abgedeckt oder offen in steriler Umgebung kultiviert.

Zur Durchführung der unterschiedlichen biokatalytischen Reaktionen ist es vielfach erforderlich während des laufenden Versuches verschiedene Flüssigkeiten oder Gase der laufenden Reaktion hinzuzuführen. Besonders hervorzuheben sind hierbei die Zufuhr von pH-Stellmitteln (Laugen und Säuren) zur pH-Titrierung der laufenden Reaktion und die Zufuhr von Substraten. Erst die Zufuhr von Substraten während des Prozesses ermöglicht die Durchführung von geregelten Batch, Fedbatch und kontinuierlichen Prozessen. Diese Betriebsmodi sind besonders wichtig für ein flexibles und erfolgreiches biologisches Screening und eine weitergehende Verfahrensentwicklung.

Im Folgenden wird kurz auf die Bedeutung der wichtigsten Betriebsweisen eingegangen:

Der pH-Wert eines Mediums ist einer der wichtigsten Umwelteinflüsse für das Zellwachstum und die Entwicklungsfähigkeit von Mikroorganismen. So werden die Aktivitäten der Enzyme, die alle stoffwechselbedingten Reaktionen katalysieren, maßgeblich vom pH-Wert beeinflusst. Der pH-Wert des Mediums verändert sich aber fortlaufend durch den Stoffwechsel der Organismen und durch den Verbrauch der Bestandteile des Kulturmediums. Bei Mangel an einer pH-Regelung ist das Erreichen hoher Zelldichten nur schwer möglich. Deshalb ist eine Regelung des pH-Wertes durch Titration von pH-Stellmittel (Lauge bzw. Säure) erforderlich. Das pH-Stellmittel kann dabei flüssig oder gasförmig sein.

In industriellen Produktionsprozessen stellt die Fedbatch Betriebsweise (Kultivierung unter Zufuhr von Substrat) den Standardprozess dar. Die Fedbatch Betriebsweise ist in vielen Produktionsprozessen notwendig, um z.B. Inhibierungen durch zu hohe Substratkonzentrationen oder Katabolitrepressionen zu vermeiden. Für die entsprechenden Bioreaktoren muss somit eine Fedbatch Betriebsweise möglich sein. Zusätzlich ist gerade in dieser Betriebsweise, durch den sich stark verändernden pH-Wert, fast immer eine pH-Regelung notwendig.

Weiterhin ist eine kontinuierliche Reaktionsführung biokatalytischer Systeme zur Erzielung eines dauerhaft konstanten Zustandes (Steady state oder Fließgleichgewicht) und zur Bestimmung kinetischer Parameter sowie zur Durchführung von Langzeitversuchen (z.B. zur Bestimmung der genetischen Stabilität von gentechnisch veränderten Mikroorganismen) wichtig.

Die Regulierung des pH-Werts sowie die Durchführung der Reaktionen im Fedbatch Betrieb ist in der Industrie im Großmaßstab seit vielen Jahrzehnten Stand der Technik. Ebenso setzen sich vermehrt kommerziell erhältliche Reaktorsysteme im Kleinmaßstab (> 10 mL - 500 mL) durch (z.B. fedbatch-Pro von DasGip [US 6202713], Sixfors von Infors etc.).

Die Reaktorsysteme im Kleinmaßstab werden für das Screening und die Prozessent-wicklung eingesetzt und können unter den oben genannten Bedingungen betrieben werden. Jedoch gilt: je kleiner das Reaktionsvolumen desto aufwendiger und kostenintensiver ist der realisierte Lösungsansatz. Aufgrund des hohen technischen Aufwandes bei kleinen Reaktionsvolumina und des niedrigen realisierbaren Parallelisierungsgrades bei höheren Reaktionsvolumina weisen die vorgeschlagenen Lösungsansätze starke Limitierungen auf. So ist mit den aktuellen Lösungsansätzen kein Hochdurchsatz-Screening (High-Throughput-Screening) (>16, >20, >50, >500, >1.000, >10.000 bis >100.000 Reaktionen pro Tag) bei gleichzeitig hoher Informationsdichte (High-Content-Screening) (>1, >2, >3, >4, >5 Reaktionsparameter pro Reaktionszeitpunkt) möglich.Für die Realisierung der beschriebenen Betriebsmodi in Mikroreaktoren Arrays sind derzeit unterschiedliche Lösungsansätze realisiert oder werden verfolgt. Hierbei kann grob in kontaktlose, kontaktbehaftete und mikrofluidische Dosierungsverfahren unter-schieden werden:

Die Technik zum kontaktlosen Hinzudosieren von sehr kleinen Fluidmengen basiert größtenteils auf der Piezotechnologie. Durch die hohen Beschleunigungen (bis zu 100.000 g), die durch ein Piezoelement erreicht werden können, ist es möglich sehr kleine Tropfen (> 30 pL) aus einem Reservoir abzutrennen und somit zu dosieren. Von diversen Firmen (z.B. Nano-Plotter^{™} von GeSim, Autodrop von Microdrop) werden hierzu Vorrichtungen zur Zudosierung von sehr kleinen Flüssigkeitsvolumen in MTP angeboten.

Weiterhin existieren kontaktlose Dosierungsverfahren die mit Druckluft (z.B. Vieweg GmbH) [EP 1036594] betrieben werden. Die realisierbaren Tropfenvolumen liegen im Bereich von zirka 5 µL bis 0,2 mL.Es bestehen auch Patente zur Kultivierung von Zellen in Mikroreaktoren unter getropfter Zufuhr von Reaktionsflüssigkeiten [DE 10221565, DE 10019862, DE 10046175]. Hierbei werden die Reaktionsflüssigkeiten durch Kapillaren bis an oder über die Reaktionskammer geleitet, bis sich an der Spitze der Kapillare ein genügend großer Tropfen gebildet hat und dieser in den Reaktionsraum hineintropft. Das Volumen eines solchen Tropfens liegt bei wässrigen Medien in der Größenordnung von ca. 20 µL und hängt vom Durchmesser und der Beschaffenheit der Kanüle ab.

Bei kontaktbehafteten Dosierverfahren erfolgt die Zufuhr der Fluide meist durch ein gesteuertes Eintauchen von Pipettenspitzen in die Reaktionsflüssigkeiten der einzelnen Kammern der Mikroreaktoren Arrays durch einen Pipettierroboter. Hierbei werden seriell (eine Pipettenspitze) oder parallel (mehrere Pipettenspitzen) in die einzelnen Kammern die gewünschten Mengen an Fluiden hinzudosiert. Durch das Eintauchen der Pipette in die Reaktionsflüssigkeit kann der dosierte Tropfen von der Pipettenspitze abgetrennt werden, so dass auch Mengen im Picoliter-Bereich dosiert werden können [DE 10201749, DE 10116642, WO 02/080822].

Auch die Nutzung des mikrofluidischen Transports in Mikroreaktoren ist nicht neu. Es existieren bereits mehrere Ansätze zum Transport und zur Steuerung kleinster Flüssigkeitsmengen in Mikroreaktoren, z.B. WO 02/37096, US 6403338, EP 1142641, DE60000109, US 6268219, WO 01/68257, US 2005/0032204, US 2005/0084421, WO 02/060582, WO 03/025113, WO01/68257, WO2004/069983.

Betrachtet man die aktuellen Lösungsansätze und bewertet diese unter den Kriteriensteriler Betrieb des Reaktionsraumes, Sauerstoffversorgung der biologischen Kulturen und Realisierung einer individuellen Zu- bzw. Abfuhr von Fluiden, müssen für die einzelnen Lösungsansätze folgende Nachteile festgehalten werden.

Durch eine kontaktlose Freistrahldosierung auf Basis der Piezotechnologie können zwar sehr kleine Fluidmengen dosiert werden, jedoch sind diese Verfahren kostenintensiv und störanfällig.

Druck- oder Schwerkraftgetriebene Verfahren sind im Vergleich zu piezotechnologiebasierten Verfahren kostengünstiger, weisen aber den Nachteil auf, dass nur vergleichsweise größere Tropfenvolumen (> 5 µL bzw. < 0,2 mL) dosiert werden können. Durch das Volumen-verhältnis der zugeführten Tropfen zum Reaktionsvolumen der einzelnen Mikrotiterplatten findet bei der Zugabe eines Tropfens von Reaktionsflüssigkeit ein extremer Sprung der entsprechenden Konzentrationen in den Reaktionskammern statt. Durch diese Konzentrationssprünge werden die Versuche stark beeinflusst, so dass die gewonnenen Versuchsergebnisse nicht repräsentativ und nicht auf größere Maßstäbe übertragbar sind. Solchen Konzentrationssprüngen kann dann nur durch eine Vergrößerung des Kulturvolumens entgegen gewirkt werden.

Ein weiterer Nachteil kontaktloser Dosierverfahren ist, dass das Verschließen der Mikroreaktoren Arrays durch eine Membran oder Deckel nicht oder nur mit sehr hohem Aufwand möglich ist. Das Verschließen ist notwendig, um die einzelnen Reaktionsgefäße einer MTP steril oder monoseptisch zu halten und die Verdampfung von Reaktionsflüssigkeit zu minimieren.

Kontaktlose Dosierverfahren ermöglichen außerdem keine Dosierung, während des kontinuierlichen Schüttelns von Mikroreaktoren Arrays. Man spricht von intermittierender Dosierung, da eine Zudosierung erst durch Anhalten des Schüttelvorgangs möglich. Eine nichtintermittierende Zudosierung ist aber bei biokatalytischen Reaktionen wünschenswert, da durch das Anhalten des Schüttelvorgangs die Betriebsbedingungen (z.B. Unterbrechung der Sauerstoffzufuhr und der Durchmischung) schlagartig geändert werden und somit derart erzielte Versuchsergebnisse nur eingeschränkt verwertet werden können.

Bei kontaktbehafteten Dosierverfahren besteht durch das Eintauchen der Pipette in die Reaktionsflüssigkeit eine hohe Gefahr an Kontaminationen oder Querkontaminationen. Außerdem wird bei diesen Verfahren ebenfalls das Verschließen der Mikroreaktoren Arrays durch eine Membran oder einen Deckel verhindert und eine Zudosierung von Flüssigkeiten während des Schüttelvorgangs ist ebenfalls nicht möglich.

Die existierenden mikrofluidischen Ansätze zum Transport und zur Steuerung kleinster Fluidmengen in Mikroreaktoren weisen mindestens einen der folgenden Mängel auf: Die Verfahren zielen nicht darauf ab, variable Volumina in einzelne Kammern von Mikroreaktoren Arrays zu dosieren. Die Verfahren dienen ausschließlich zur Analytik von Stoffen und Stoffströmen, zur Auftrennung von Stoffströmen und/oder zur Bereitung möglichst kleiner Probenvolumina. Die Verfahren erlauben nur eine gleichmäßige Aufteilung von Fluidvolumina in Mikroreaktoren Arrays. Ein gezieltes Zudosieren in einzelne Kammern ist damit nicht möglich. Die Verfahren erlauben keinen sterilen Verschluss der Mikroreaktoren Arrays durch eine Membran oder einen Deckel. Die Verfahren erlauben nicht das Zudosieren oder Abführen von Fluiden während des kontinuierlichen Schüttelns der Mikroreaktoren. Arrays zur Durchmischung der Reaktionsflüssigkeiten und zur Einstellung ausreichender Stofftransferraten.

Zusammenfassend kann festgehalten werden, dass alle Lösungsansätze es nicht ermöglichen folgenden Anforderungen an einen biotechnologischen, biochemischen oder chemischen Prozess zu vereinen:
1. eine individuelle Zu- bzw. Abfuhr von Fluiden in die einzelnen Reaktionskammern eines Mikroreaktoren Arrays (z.B. zur Realisierung einer pH-Titrierung oder eines Fedbatch Prozesses);
2. ein steriler oder monoseptischer Verschluss des Mikroreaktoren Arrays während der Zu- oder Abfuhr von Fluiden;
3. einen ausreichenden und kontrollierten Stofftransfer (z.B. zur Sauerstoffversorgung) durch Schütteln.

Die US 2003/008308 A1 beschreibt zwar einen Mikroreaktorenarray mit Pumpen und vielen Leitungen. Beispielsweise für eine pH-Regulierung in einem geschüttelten Behälter ist diese Vorrichtung jedoch nicht geeignet.

Ausgehend von diesem Stand der Technik liegt der Erfindung die Aufgabe zu Grunde, zumindest einige dieser Einschränkungen für Mikroreaktoren Arrays zu beseitigen. Denn bereits im kleinsten Maßstab des Primärscreenings sollten möglichst ähnliche Betriebsbedingungen herrschen wie in einer späteren Produktion, da sonst eine Selektion nach den falschen Kriterien erfolgt. Eine fehlerhafte Auswahl von Katalysatoren (Stämme, Enzyme, Proteine oder chem. Katalysatoren) im Primärscreening aufgrund ungeeigneter Testbedingungen wirkt sich auf die gesamte folgende Prozessentwicklung aus und kann in der Regel nicht mehr kompensiert werden. Die wirtschaftlichen Nachteile, die erheblich sein dürften, lassen sich allerdings nicht quantifizieren, da sich bis heute nicht die geeigneten Techniken im Mikromaßstab boten, die z.B. eine pH-Regelung und Fedbatch Betriebsweise im Primärscreening möglich machten.

Um solche Betriebsweisen in Mikroreaktoren Arrays zu realisieren, muss aufgrund der kleinen Reaktionsvolumina die Zu- und Abfuhr der Fluidmengen im Nano- oder Pikoliterbereich erfolgen. Dies ist notwendig, um die Prozesse angemessen zu beeinflussen oder zu regeln und um ggf. dieselben Verhältnisse von zudosiertem bzw. abgeführtem Volumen zu Reaktionsvolumen wie in größeren Reaktoren zu erreichen. Hierdurch kann eine Übertragbarkeit der Versuchsergebnisse auf größere Maßstäbe (scale-up) ermöglicht werden.

Die Aufgabe wird gelöst durch eine Vorrichtung mit den Merkmalen des Patentanspruchs 1.

Die erfindungsgemäße Vorrichtung ermöglicht ein Fluidsystem zur Zu- und Abfuhr von Fluiden. Ein Fluidsystem wird definiert als eine Vorrichtung bestehend aus einem Kanalsystem, das verschiedene Kanäle auf einer oder mehreren Ebenen vorhält, durch die die gewünschten Fluide transportiert werden. Des Weiteren verfügt das Fluidsystem vorzugsweise über Ventile, die die einzelnen Kanäle verschließen können und somit eine Steuerung der Fluidkanäle erlauben. Sind die Kanäle zum Fluidtransport und die Aktorik oder Ansteuerung der Ventile auf unterschiedlichen Ebenen oder Substraten realisiert, werden diese Ebenen als Steuerungsebene bezeichnet. Durch die Fluidkanäle werden die Fluide individuell in die einzelnen Reaktionskammern transportiert. Durch einen gleichzeitigen Schüttelvorgang des Mikroreaktoren-Arrays können sich die in die Reaktionskammern transportierten Fluidmengen mit den übrigen Reaktionsvolumen vermischen. Durch eine Steuerung der einzelnen Zuläufe in die unterschiedlichen Reaktionskammern des Mikroreaktoren Arrays kann ein individueller Zu- und Ablauf realisiert werden.

Die Steuerung der einzelnen Fluidkanäle kann durch unterschiedliche Verfahren realisiert werden. Angedacht sind hier unter anderem pneumatische Steuerkanäle, die rechtwinklig zu den Fluidkanälen auf einer anderen Ebene verlaufen und bei Druckbeaufschlagung den gewünschten Fluidkanal durch eine dünne Membran abklemmen und schließen können. Aber auch optisch, thermisch, elektromechanisch oder magnetisch aktuierte Mikroventile können zur Fluidkanalsteuerung verwendet werden. Um eine Diffusion der Reaktionsmedien innerhalb des Reaktors zurück in die Fluidkanäle zu minimieren, können einfache passive Ventile am Einlass des Kanals in den Reaktor realisiert werden.

Zusätzlich können die Fluidkanäle und Reaktorkammern derart ausgelegt werden, dass sie zumindest teilweise durchlässig für elektromagnetische Strahlung (wie z.B. Licht) sind. Hierdurch werden elektromagnetische oder nicht-invasive optische Reaktionsüberwachungen ermöglicht. Das Fluidsystem kann je nach Messaufgabe als Deckel, Boden oder aber auch als Mittelebene des Mikroreaktoren Arrays ausgeführt werden, so dass der Zugang zu den Mikroreaktoren von oben oder unten gewährleistet ist.

Die Erfindung bietet in ihren unterschiedlichen Ausgestaltungen folgende Vorteile gegenüber bisher realisierten Lösungsansätzen:

Es wird die individuelle Zu- und Abfuhr von Reaktionsfluiden in die einzelnen Kammern von Mikroreaktoren Arrays ermöglicht.

Die Zu- und Abfuhr von Reaktionsfluiden kann so realisiert werden, dass ein optischer Zugang zu jeder einzelnen Kammer des Mikroreaktoren Arrays gewährleistet bleibt. Dadurch können in jeder einzelnen Kammer durch optische Messungen nicht-invasiv die Betriebszustände ermittelt werden.

Die individuelle Zudosierung und Messdatenerfassung kann die Regelung von Stellgrößen für jede einzelne Reaktionskammer ermöglichen.

Die Zudosierung kann erfolgen ohne den Schüttelvorgang unterbrechen zu müssen.

Die Reaktionskammern sind mit einer gasdurchlässigen Membran oder einem gasdurchlässigen Deckel verschlossen. Dies ermöglicht einen sterilen Betrieb und minimiert die Verdampfung, wodurch das Reaktionsvolumen weitgehend konstant gehalten werden kann.

Im Hinblick auf den Stand der Technik werden somit folgende Nachteile durch die erfindungsgemäße Anordnung und durch Weiterbildungen dieser Anordnung beseitigt:

Die für die einzelnen Reaktionskammern benötigten sehr kleinen Mengen (> 10 pL) an Fluiden (z.B. pH-Stellmittel, Substrat) können in die einzelnen Kammern steril zu- bzw. abgeführt werden.

Die Reaktionskammern sind durch eine semipermeable Membran oder einen Deckel steril verschlossen.

Die Zugabe der Fluide kann ohne einen eventuellen Schüttelvorgang zu unterbrechen erfolgen. Die Sauerstoffversorgung der sich in den Kammern befindlichen Organismen wird somit nicht unterbrochen und eine kontinuierliche gute Durchmischung der Flüssigkeiten, Feststoffe und Gase wird gewährleistet.

Das System kann bezüglich Form und Anwendbarkeit auf kommerziell erhältlichen Mikrotiterplatten basieren.

Die in den Ansprüchen beschriebenen Verfahren und Vorrichtungen zeigen weitere Merkmalskombinationen auf, die für sich erfindungswesentlich sind und die auch in jeder weiteren Kombination untereinander und auch in Verbindung mit zuvor genannten Merkmalen erfindungswesentliche Aspekte beschreiben.

Erfindungsgemäß weist bei den Mikroreaktoren-Arrays jedes Behältnis einen Kanal auf. Dadurch können die unterschiedlichen Behältnisse individuell versorgt werden. Insbesondere zur Zu- und Abfuhr von Fluid kann es vorteilhaft sein, die Behältnisse mit mindestens zwei Kanälen zu verbinden.

Dadurch entsteht ein Mikroreaktoren-Array bei dem ein oder mehrere Fluidkanäle in jeden einzelnen Mikroreaktor führen. Das Mikroreaktoren-Array kann dadurch individuell gesteuert und/oder geregelt werden. Dadurch wird eine Prozesssteuerung, -regelung, - beeinflussung und/oder Prozesskontrolle möglich.

Ein Ausführungsbeispiel sieht vor, dass die Behältnisse in einer Ebene angeordnet sind und der Kanal in der Ebene der Behältnisse angeordnet ist. Hierzu kann der Kanal beispielsweise in den Seitenwänden des Mikroreaktoren-Arrays realisiert werden. Die zudosierten Fluide stehen dadurch nicht mehr in ständigem Kontakt mit der Flüssigkeit in den Mikroreaktoren. Der durch den Fluidkanal präsentierte Tropfen wird dabei durch die durch das Schütteln erzeugte Flüssigkeitssichel von der Seitenwand abgetrennt und von der Reaktionsflüssigkeit aufgenommen.

Eine andere Ausführungsvariante sieht vor, dass die Behältnisse in einer Ebene angeordnet sind und der Kanal unter der Ebene der Behältnisse angeordnet ist.

Insbesondere, wenn der Mikroreaktoren-Array einen Deckel aufweist, ist es vorteilhaft, wenn die Behältnisse in einer Ebene angeordnet sind und der Kanal über der Ebene der Behältnisse angeordnet ist. Die Fluidkanäle können somit im Deckel des Mikroreaktoren-Arrays realisiert werden und die zu dosierenden Fluide stehen dadurch nicht im ständigen Kontakt mit der Flüssigkeit in den Mikroreaktoren. Der durch den Fluidkanal angebotene Tropfen wird auch bei dieser Ausführungsform durch die durch das Schütteln erzeugte bis zum Deckel reichende Flüssigkeitssichel von dem Deckel gelöst und in die Reaktionsflüssigkeit aufgenommen.

Der Mikroreaktoren-Array kann auch so aufgebaut sein, dass die Fluidkanäle zur Zufuhr von Fluiden im Deckel des Mikroreaktoren-Arrays realisiert sind und die Ausgänge der Fluidkanäle nicht in Kontakt mit der Flüssigkeit in den Mikroreaktoren stehen. Die Fluidtropfen werden dann durch hohe Beschleunigungen gelöst und der Reaktionsflüssigkeit zugeführt.

Verständlicherweise können auch unterschiedliche Kanäle in unterschiedlichen Positionen relativ zur Ebene der Behältnisse angeordnet sein.

Zur Realisierung unterschiedlicher Mikroreaktoren-Arrays wird daher vorteilhafterweise vorgeschlagen, dass der Übergang zwischen Kanal und Behältnis oberhalb der Befüllebene des Behältnises angeordnet ist und der Array eine Abdeckung aufweist, in der der Kanal angeordnet ist.

Erfindungsgemäß weist der Mikroreaktoren-Array eine Pumpe auf. Ein Ventil ermöglicht eine gesteuerte Zuführung von Fluiden und eine Pumpe ermöglicht die notwendige Druckerhöhung, um Fluide in die Reaktionskammern zu befördern. Die gezeigten Ausführungsbeispiele erläutern, dass ein Ventil auch auf einfache Art und Weise zu einer Pumpe weiterentwickelt werden kann.

Zur Ansteuerung des Ventils oder einer Pumpe wird ein fokussierter Lichtstrahl, eine thermische Ansteuerung oder eine Ansteuerung durch Mirkowellen vorgeschlagen. Ein Lichtstrahl kann auch über insbesondere in die Steuerungsebene integrierte Lichtleiter auf die jeweiligen Ventile fokussiert oder geleitet werden. Eine thermische Anregung ist beispielsweise durch eine elektrische Heizwendel zu realisieren. Weitere Möglichkeiten erschließen sich durch magnetisch oder induktiv angesteuerte Ventile und Pumpen.

Außerdem kann der Kanal durch Übergänge von hydrophilen und hydrophoben Oberflächen eine Ventilfunktion übernehmen. Dadurch entstehen passive Ventile zur Steuerung der Fluide.

Es wird vorgeschlagen, dass der Kanal thermisch oder elektrisch formverändernde Materialen aufweist. Hierzu können thermisch formverändemde Polymere und/oder Metall-Legierungen verwendet werden. Außerdem können auch Formgedächtnis-Legierungen (SMA) und Formgedächtnispolymere sowie elektrisch formveränderbare Keramiken (Piezo- Kristalle) zur Realisierung von Ventilen und Pumpen eingesetzt werden.

Ein Ausführungsbeispiel sieht vor, dass der Kanal eine durch Druck verformbare Wandung als Ventil oder Pumpe aufweist. Durch einen gesteuerten pneumatischen oder mechanischen Druck von außen kann eine der Seiten des Fluidkanals in diesen hineingedrückt werden um ihn zu verschließen und um dadurch eine aktive Ventilfunktion oder auch eine Pumpfunktion zur realisieren.

Die im Fluidsystem verwendeten Ventile oder Pumpen können auch eine Folie oder ein dünnes Substrat aufweisen, welches mit einer elektrischen Schaltung zusammenwirkt, die als Steuerebene des Fluidsystems eingesetzt wird.

Eine Ausgestaltung sieht vor, dass Vertiefungen auf dem Array als pneumatische Anschlüsse ausgebildet sind. Derartige Vertiefungen und vorzugsweise eine oder mehrere Reaktionskammern insbesondere einer Mikrotiterplatte können auf einem Mikroreaktoren-Array als pneumatische Anschlüsse zur Steuerung der Fluidströme genutzt werden.

Insbesondere bei Verwendung eines Drucks auf eine verformbare Wandung kann bei mehrfacher Hintereinanderschaltung ein peristaltischer Pumpeffekt erzeugt werden, wobei der Druck auf einer Seite des Fluidkanals pneumatisch oder mechanisch erzeugt werden kann.

Insbesondere wenn die Fluidkänale im Boden des Mikroreaktoren-Arrays ausgeführt sind, können die zu dosierenden Fluide durch ein Ventil getrennt mit der Reaktionsflüssigkeit in den Mikroreaktoren in Kontakt stehen. Eine Alternative sieht vor, dass die zu dosierenden Fluide mit einer teildurchlässigen Membran von der Reaktionsflüssigkeit in den Mikroreaktoren getrennt werden. Hierzu wird im Kanal oder im Behältnis eine teildurchlässige Membran angeordnet.

Vorteilhaft ist es, wenn mindestens eine Fläche des Arrays als optischer Zugang ausgebildet ist. Durch die Verwendung geeigneter Materialien kann mindestens eine Fläche der einzelnen Mikroreaktoren als optischer Zugang die individuelle Erfassung von Messdaten aus den einzelnen Mikroreaktoren ermöglichen, die zur Regelung der Prozesse dienen können.

Außerdem ist es vorteilhaft, wenn das Mikroreaktoren-Array Sensoren in den einzelnen Behältnissen zur Erfassung von Messdaten aufweist. Hierbei können unterschiedliche Sensoren wie insbesondere auch chemische Sensoren in gelöster oder immobilisierter Form in den einzelnen Mikroreaktoren zur individuellen Erfassung von Messdaten eingesetzt werden.

Ein Ausführungsbeispiel sieht vor, dass Behältnisse des Mikroreaktoren-Arrays eine Form oder Oberflächenstruktur aufweisen, die dazu geeignet ist, einen Kontakt zwischen Flüssigkeit und Kanal zu minimieren. Dadurch dass eine spezielle Form der einzelnen Mikroreaktoren oder in ihnen enthaltene Zusatzstrukturen den Kontakt von Flüssigkeitssichel und Fluidkanalausgang minimieren, kann eine Diffusion der Flüssigkeit in die Kanäle verhindert werden.

Die der Erfindung zu Grunde liegende Aufgabe wird von einer Vorrichtung mit einem Mikroreaktoren-Array gelöst, die einen mechanischen Schüttler aufweist.

Vorteilhaft ist es, wenn die Bewegungseinrichtung mindestens eine in den Mikroreaktoren-Array integrierte Pumpe antreibt. Eine derartige Pumpe kann den Druck in einem Fluidkanal erhöhen, der nach Öffnen eines Ventils für den Fluidtransport sorgt.

Eine Ausgestaltung sieht vor, dass der Kanal so ausgebildet und relativ zu einem Behältnis mit Reaktionsflüssigkeit angeordnet ist, dass eine Fluidmenge am Kanalende nur beim Bewegen der Reaktionsflüssigkeit durch die Sichel der Flüssigkeitsoberfläche abgenommen wird.

Vorteilhaft ist es, wenn die Vorrichtung eine Mikrotiterplatte und eine Steuerungsebene mit Ventilen aufweist. Die Steuerungsebene kann neben Ventilen zur Kontrolle von Durchflüssen auch Pumpen für den eigentlichen Fluidtransport beinhalten. Anderseits kann die Steuerungsebene auch nur Ventile zur Kontrolle von Durchflüssen beinhalten, wobei der eigentliche Stofftransport durch externe Pumpen oder Druckbeaufschlagung realisiert wird.

Die Aufteilung von Mikrotiterplatte und Steuerungsebene ermöglicht es, Mikroreaktoren-Arrays zu verwenden, die auf dem Standartformat von Mikrotiterplatten basieren. Die Mikrotiterplatte kann dadurch durch ein fluidisches Zudosierungssystem erweitert werden. Anderseits kann eine Mikrotiterplatte auch selbst einen Teil des Fluidsystems darstellen und nur durch eine Steuerungsebene erweitert sein. Vorteilhaft ist es, wenn die Steuerungsebene eine elektrische Schaltung aufweist.

Außerdem wird vorgeschlagen, dass eine Fluidebene und eine Steuerungsebene modular kombiniert sind. Dadurch wird eine Widerverwendung der komplexen Steuerungsebene gewährleistet, während die Fluidebene als steriles Einmalprodukt hergestellt werden kann.

Die der Erfindung zu Grunde liegende Aufgabe wird auch durch ein Verfahren zur Verwendung einer derartigen Vorrichtung mit den Merkmalen des Patentanspruchs 16 gelöst. Das Vorsehen der Fluidkanäle ermöglicht es, die Fluidkanäle individuell zu steuern oder zu regeln. Außerdem können die zu- oder abgeführten Fluidmengen während eines Mischvorgangs in das Volumen der Reaktionsflüssigkeit ein- oder abgeleitet werden. Ein kontinuierlicher Schüttelvorgang sorgt für einen ausreichenden Stofftransport und die Durchmischung der Reaktionspartner oder der Fluide. Die Reaktion der Reaktionspartner wird hierbei nicht durch die Stofftransferbedingungen limitiert oder eingeschränkt.

Eine Ausgestaltung des Verfahrens sieht vor, dass eine Fluidmenge am Kanal vorgehalten wird und nur beim Bewegen der Reaktionsflüssigkeit durch die Sichel der Flüssigkeitsoberfläche abgenommen wird.

Eine besondere Verfahrensführung sieht vor, dass in einem ersten Schritt die benötigten Fluidmengen in einer dafür vorgesehenen Fluidvorhaltekammer mittels herkömmlicher Dosierungsverfahren wie Mikro- oder Nanodispenser eingefüllt werden und in einem zweiten darauf folgenden Schritt die Zugänge für die Dosierung druckdicht versiegelt werden und die vordosierten Fluidmengen mit einem Überdruck durch Membranen hindurch in die Mikroreaktoren gedrückt werden. Bei einer speziellen Verfahrensführung ist der Reaktionsraum bis zum Ende der Reaktion der Reaktionspartner in jedem Mikroreaktor zu jedem Zeitpunkt entweder durch eine Membran oder einen Deckel verschlossen, um somit einen unkontrollierten Ein- oder Austrag von Materie oder Kontaminationen zu vermeiden.

Wenn der Mikroreaktoren-Array mit einer Membran oder einem Deckel abgedeckt und alle zu- und abführenden Fluide durch vorsterilisierte Materialien geleitet werden, kann die gesamte Reaktionsführung steril oder monoseptisch gehalten werden.

Außerdem können die zuzuführenden Fluide in einer Vertiefung vorzugsweise einer der Reaktionskammern auf dem Mikroreaktoren-Array oder einem weiteren mit Zuleitungen verbunden Vorlagegefäß vorgehalten werden. Die zuzuführenden Fluide können auch in einem separaten Behältnis vorgehalten werden, welches nicht mitgeschüttelt wird, um sie dann über Kanäle oder Schläuche zu den einzelnen Mikroreaktoren zu führen.

Die Figuren zeigen jeweils in schematischer Darstellung Ausführungsbeispiele der Erfindung, weitere Erfindungen und erfinderische Merkmalskombinationen.

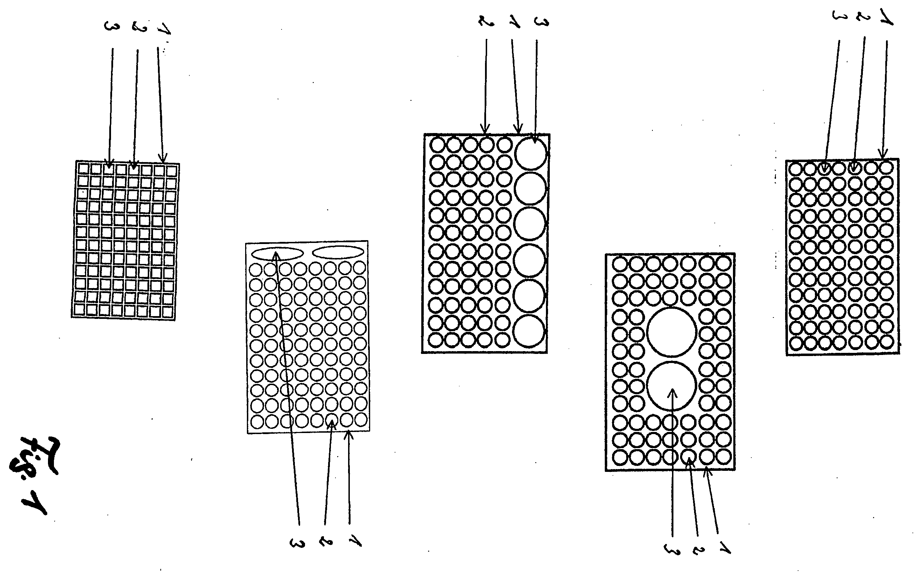

Die Figur 1 zeigt verschiedene Möglichkeiten der Realisierung eines Mikroreaktor Arrays 1, das auf einem Standard-Mikrotiterplatten-Format basiert. Die einzelnen Reaktorgeometrien 2 sind dabei nicht zwingend rund zu wählen. Die Verwendung des standardisierten Mikrotiterplattenformats erlaubt uneingeschränkte Kompatibilität mit herkömmlichen Pipetierrobotern. Auf der Platte können auch Reservoire 3 realisiert werden, die die zuzuführenden Fluide vorhalten. Neben unterschiedlichen Formen für Reservoir 3 und Mikroreaktor 2 können diese ebenso formidentisch sein. So können z.B. in bestehende Mikroreaktoren Arrays einige Mikroreaktoren 2 als Reservoire 3 für die zu transportierenden Fluide genutzt werden. Diese Reservoire 3 sind in ihrer Form und Position beliebig auf der Mikrotiterplatte herzustellen.

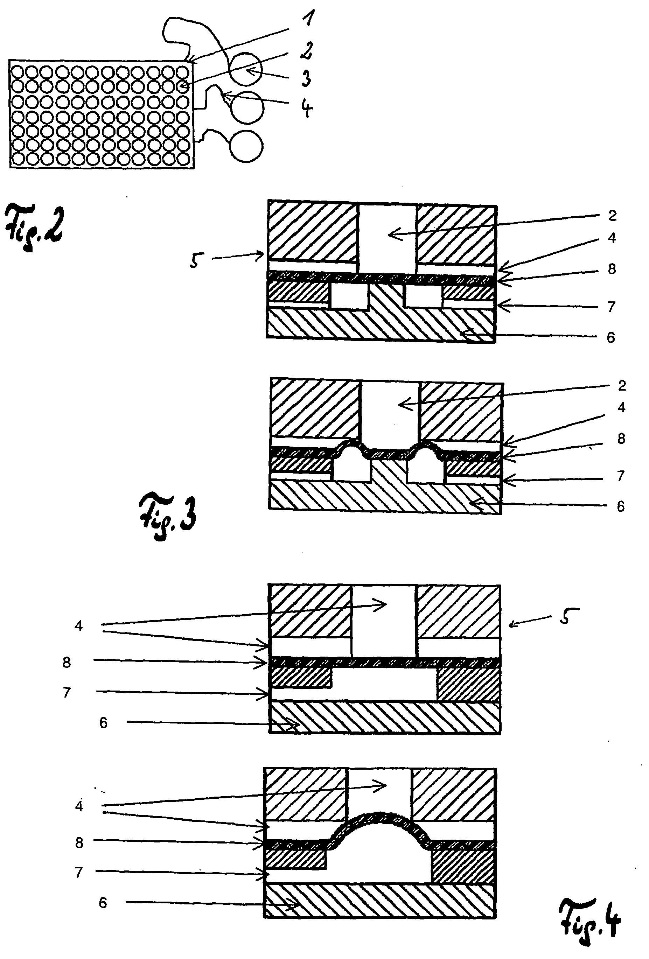

In manchen Fällen kann es sinnvoll sein, die Fluide, die den Reaktoren 2 zugeführt oder aus diesen abgeführt werden sollen, in separaten nicht mitgeschüttelten Reservoiren 3 vorzuhalten. Diese sind dann über Schläuche oder flexible Fluidkanäle 4 wie in Figur 2 gezeigt mit den Reaktoren des Mikroreaktor Arrays verbunden.

Das Verschließen einzelner oder mehrerer Fluidkanäle 4, die in einen Reaktor 2 führen, kann - wie in Figur 3 gezeigt - durch dünne Membranen 8 realisiert werden, die durch einen pneumatischen Kanal 7 mit einem Luftdruck beaufschlagt werden, sich in den Fluidkanal 4 drücken und diesen damit verschließen. Die Fluidebene 5 und die pneumatisch aktuierte Steuerungsebene 6 sind im gezeigten Beispiel Teil des Bodens des Mikroreaktor Arrays 1. Die hier gezeigten Ventile dienen nur der Steuerung. Der eigentliche Stofftransport kann durch externe Pumpvorrichtungen realisiert werden.

Die Figur 4 zeigt, wie durch geschickte Anordnung von Fluidkanälen 4 der Fluidebene 5 und der pneumatisch aktuierten 7 Membranen 8 der Flüssigkeits- oder Gasdruck in einer Abzweigung des Fluidkanals 4 erhöht werden kann und gleichzeitig weitere Zuführungskanäle geschlossen werden können. So kann durch eine einzige Membran 8 in nur einem Schritt ein Kanal mit einem Druck beaufschlagt werden während andere Fluidkanäle 4 geschlossen werden.

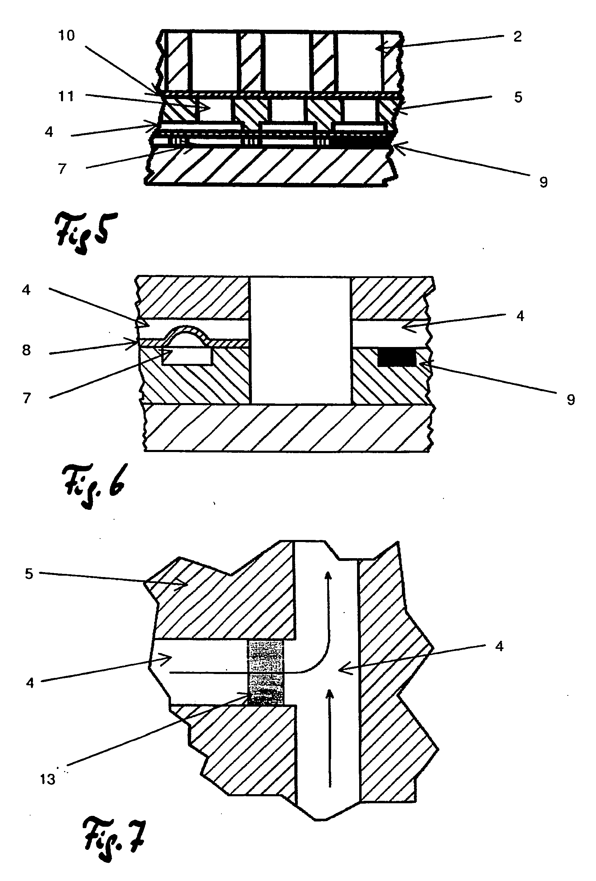

Die Verwendung einer semipermeablen Membran 10 am Übergang von Fluidkanal 4 zu Reaktionskammer 2 zeigt die Figur 5. Sie kann auf einfache Art und Weise einen unkontrollierten diffusiven Stofftransfer von Kanal zu Reaktor und umgekehrt minimieren. Ein Aktuator 9 (z.B. Piezokristall, Formgedächtnislegierung, formveränderliche Polymere, etc.) oder ein durch eine Membran getrennter Pneumatikkanal 7 bringt die Pumpleistung auf, die benötigt wird, um das zuzuführende Medium in der Fluidvorkammer 11 durch die Membran 10 in die Reaktionskammer 2 zu drücken.

Neben der Realisierung einer Kanalzuführung 4 im Boden kann auch eine in Figur 7 gezeigte Seitenwandzuführung sinnvoll sein. Um einen Tropfen an der Innenseite des Reaktors 2 anzubieten, damit dieser von der durch das Schütteln erzeugten Sichel mittransportiert wird, können auch hier wieder die bereits erwähnten Ventil- bzw. Pumptechniken verwendet werden. Die Fluidkanäle lassen sich auch bei dieser Kanalanordnung 4 über pneumatische Steuerungskanäle oder andere Aktorik 9 (z.B. Formgedächtnislegierungen oder formveränderliche Polymere etc.) steuern.

Eine weitere Möglichkeit die Fluidströme innerhalb der Fluidebene zu -steuern bietet eine gezielte Oberflächenbehandlung der Kanäle 4. Durch den Übergang von hydrophilen und hydrophoben Oberflächen lassen sich einfache Barrieren für den Fluidstrom erzeugen. Bei dem gezeigten Beispiel wird das Fluid aus dem Seitenkanal 4 durch solch eine Barriere 13 daran gehindert in den senkrecht verlaufenden Hauptkanal 4 hinein zu fließen. Durch eine kurze Druckbeaufschlagung kann diese Barriere 13 überwunden werden. Ist es einmal zu einem Kontakt mit dem Hauptstrom gekommen, kann nur eine kurze Unterbrechung des Hauptstromes (z.B. durch Luftblasen) die Funktion der Barriere 13 wieder herstellen. Ein zusätzlicher Lufteinlass an der Stelle der Barriere kann ebenfalls dazu führen, dass der Zustrom aus dem Seitenkanal nach Druckabfall wieder zusammenbricht.

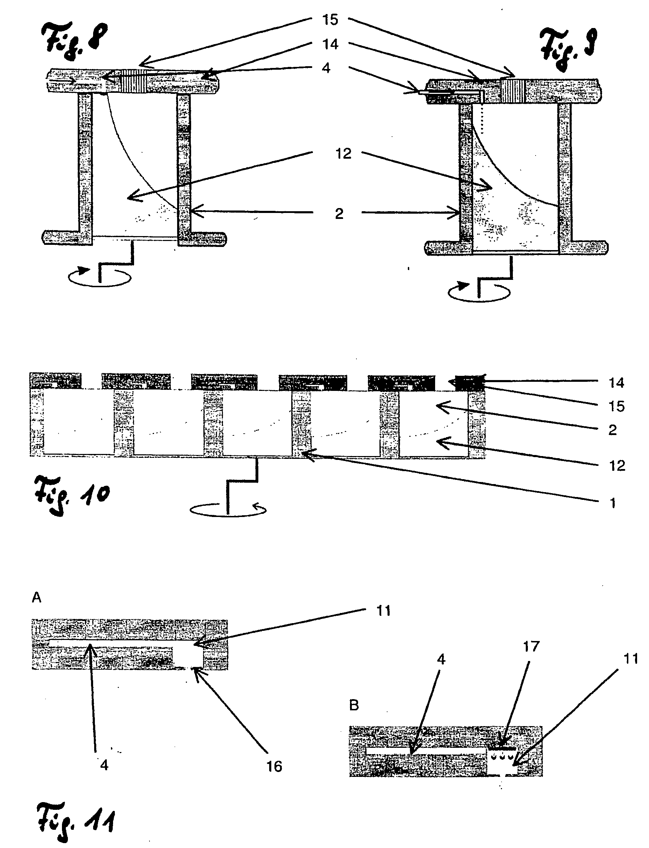

Bei einer weiteren Variante der Mikroreaktoren wird das zu dosierende Fluid wie in Figur 8 gezeigt durch eine Öffnung im Deckel angeboten. Der Deckel beinhaltet neben den Fluidkanälen 4, durch die das Fluid zu den einzelnen Reaktoren 2 transportiert wird, auch Belüftungseinlässe mit speziellen Membraneinsätzen 15, um für eine sterile Gaszufuhr zu sorgen, falls diese für den Prozess benötigt wird. Damit der durch den Kanal 4 angebotene Tropfen auch in kleinen Mengen von der Flüssigkeit aufgenommen werden kann, muss die durch das Schütteln erzeugte Flüssigkeitssichel 12 bis zum Fluidkanaleinlass reichen.

Für den in Figur 9 gezeigten Fall, dass die Flüssigkeitssichel 12 nicht bis zum Deckel reicht, müssen die aus dem Fluidkanal 4 abgelösten Tropfen stark beschleunigt werden, um das Volumen der Tropfen möglichst klein zu halten. Die zuvor erwähnten Nachteile der kontaktlosen Dosierungsverfahren werden bei diesem Ansatz umgangen. Hier ist die Dosiereinheit Teil des Deckels und kann mit dem Mikroreaktoren Array mitgeschüttelt werden. Auch hierfür muss der Deckel Fluidkanäle 4 und Belüftungszugänge 15 zu den einzelnen Reaktoren aufweisen.

Wie in Figur 10 gezeigt kann ein Deckel auf diese Weise beliebig viele Mikroreaktoren individuell mit kleinsten Fluidmengen versorgen. Die einzelnen Fluidvorkammern 11 füllen sich wie in Figur 11 gezeigt durch Kapillarkräfte. Aufgrund der nur sehr kleinen Öffnung zur Reaktionskammer 2 lösen sich durch die Oberflächenspannung keine Tropfen ab. Erst durch eine schnelle Aktuation, die das Volumen der Fluidvorkammer 11 reduziert, werden kleinste Tropfen in die Reaktionskammer geschossen. Diese Volumenreduzierung kann durch die in Figur 11 A gezeigten Heizelemente 16 oder durch die in Figur 11 B gezeigten Piezokristalle 17 realisiert werden.

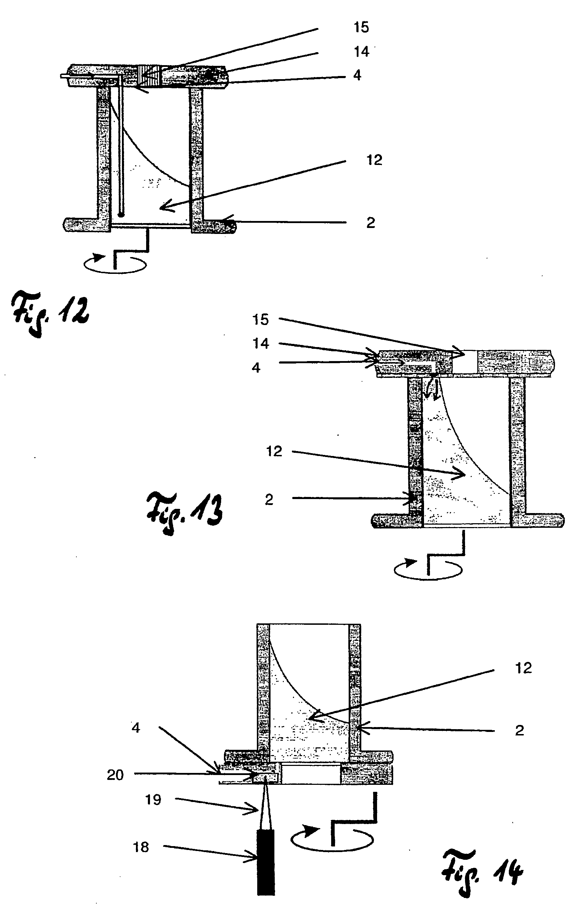

Anstelle von stark beschleunigten Tropfen zur Dosierung kleinster Fluidmengen kann auch eine Kanalverlängerung 4 aus dem Deckel heraus in die Flüssigkeit 12 innerhalb des Reaktors 2 ragen und dort, wie in Figur 12 gezeigt, in ständigem Kontakt mit der Flüssigkeit 12 für eine Zu- bzw. Abfuhr weiterer Medien sorgen. Eine ungewollte Diffusion in den Fluidkanal 4 hinein oder aus ihm heraus kann durch eine sehr kleine Öffnung oder eine semipermeable Membran weitestgehend minimiert werden.

Eine auf der Unterseite des Deckels angebrachte semipermeable Membran 10 kann in doppelter Hinsicht verwendet werden. Wie in Figur 13 gezeigt sorgt sie auf der einen Seite für einen sterilen Sauerstoffaustausch an den Belüftungseinlässen 15 und auf der anderen Seite kann durch sie auch die Fluidzufuhr durch den Fluidkanal 4 besser kontrolliert werden. Sie verhindert eine diffuse Durchmischung im Fluidkanal 4 und lässt nur Fluide hindurch, wenn der Kanal mit einem leichten Überdruck beaufschlagt wird.

Manche Materialien wie feste Polymere oder gekapselte Polymergele oder Metalllegierungen, die zur Aktuation, also als Pumpe oder Ventil verwendet werden können, lassen sich durch elektromagnetische Wellen wie Laserlicht beeinflussen und steuern 20. Das in Figur 14 gezeigte Beispiel beschreibt eine Anordnung, bei der der Mikroreaktor Array 1 mit seinen einzelnen Reaktoren 2 von der Laserlichtquelle 18 entkoppelt ist. Der Mikroreaktor Array 1 wird geschüttelt, während der ortsfeste Laser 18 auf einen bestimmten Punkt fokussiert wird. Um den zu aktuierenden Bereich durch das Licht zu aktivieren, kann entweder der Schüttelradius so klein gewählt werden, dass der zu treffende Bereich nie ganz den Fokus verlässt, oder er während der Schüttelbewegung immer durch den Laserfokus 19 geführt wird. Damit kann zwar keine kontinuierliche Bestrahlung realisiert werden, in den meisten Fällen reicht jedoch ein gepulster Energieeintrag völlig aus, um die gewünschten Effekte zu erzielen.

Ein anderer Ansatz besteht darin, die Dosierung und die Zuführung von Fluiden in die einzelnen Reaktoren 2 in zwei voneinander unabhängigen Schritten durchzuführen. Die Figur 15 A zeigt, wie in einem ersten Schritt die jeweils benötigte Fluidmenge durch einen in der x-y-Ebene verfahrbaren Mikro- oder Nanodispenser (z.B. Pipetierrobotor) 22 in die entsprechenden Fluidvorkammern 111 eingefüllt wird. Die Dosieröffnungen 21 sollten dabei so groß gewählt werden, dass der Dispenser als ortsfeste Dosierungseinheit 22 die Schüttelbewegung des Mikroreaktoren Arrays 1 nicht nachvollziehen muss. Durch eine Mechanik werden die Dosierungsöffnungen 21 nach Befüllung geschlossen und über ein pneumatisches System 7 wird wie in Figur 15 B gezeigt ein Druck auf die Fluidkanäle 4 und Fluidvorkammern 11 aufgebracht. Dieser sorgt für den Fluidtransfer durch eine semipermeable Membran 10 hindurch. Durch die Membran (Porengröße < 0,2µm) 10 wird für einen sterilen Fluideintrag gesorgt.

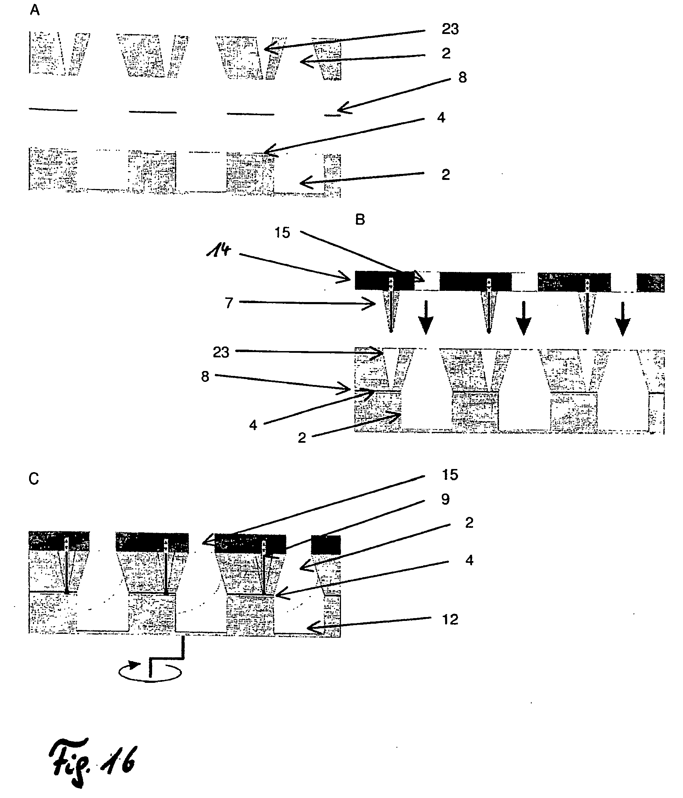

Zur kommerziellen Anwendung bietet sich ein System an, bei dem die sterilen Fluidkanäle 4 und die Aktuatoren in voneinander getrennten Systemen realisiert sind. Der Vorteil ergibt sich aus der Wiederverwendbarkeit der teuren Aktorik, während die Fluidkanäle und die Reaktoren als sterile Einmalprodukte eingesetzt werden können. Der in Figur 16 A gezeigte Aufbau der Fluid- und Reaktor-Ebene besteht aus drei Komponenten: Einem Boden mit den Reaktorkammern 2 und den Fluidkanälen 4, einer dünnen flexiblen Membran 8 mit Aussparungen an den Stellen der Reaktoren 2 und einem Aufsatz, der direkte Zugänge zu den Reaktoren 2 enthält, um diese zu befüllen und eine Sauerstoffzufuhr während der Versuche zu gewährleisten. Zusätzlich enthält der Aufsatz auch Zugänge 23 zu der Membran 8 über den Fluidkanälen 4. Diese in Figur 16 B gezeigten Zugänge 23 werden genutzt, um die an einem Deckel 14 angebrachte Aktorik 9 mit der Membran in Verbindung zu bringen. Durch konisch zulaufende Zugänge wird eine einfache Selbstjustage des Deckels 14 gewährleistet. Die im Deckel befindliche Aktorik erlaubt es, die Membran in den darunter liegenden Fluidkanal 4 zu drücken und diesen so zu verschließen. Das Fluidsystem 4 befüllt sich zunächst durch Kapillarkräfte und wird nach Verschließen der Zugänge zu den einzelnen Reaktoren 2 mit einem Überdruck beaufschlagt. Dieser Druck kann im Deckel selbst oder durch externe Pumpen erzeugt werden. Durch kurzes Öffnen der Fluidkanäle 4 entsteht ein kleiner Tropfen an der Reaktorwand. Dieser wird von der in Figur 16 C gezeigten Flüssigkeitssichel 12 abgeschert und mitgenommen.

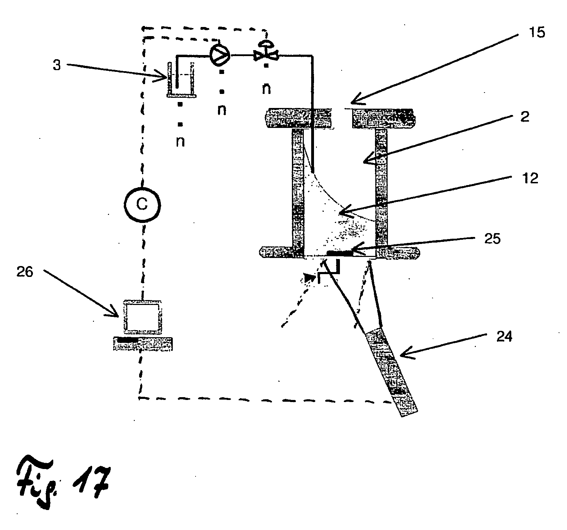

In Figur 17 ist schematisch das Regelprinzip dargestellt, das zum Beispiel für eine pHgeregelte Versuchsführung geeignet wäre. Die geschüttelten Reaktoren 2 können einzeln von außen nicht-invasiv mittels optischer Messmethoden 24 bezüglich ihres pH-Wertes ausgelesen werden. Hierzu können so genannte Optoden 25 verwendet werden. Der gemessene Wert kann kontinuierlich erfasst und ausgewertet werden. Über einen Regelkreis wird dann die Zufuhr entsprechender Stellmittel kontrolliert. Diese Lösung ist in gleicher Weise für eine Substratoder andere Substanz-Zufuhr und Fluidabfuhr realisierbar. In diesem Falle können alle aus dem Reaktionssystem optisch oder elektrisch erfassten Prozessparameter (wie z.B. pO2, pCO2, T, Biomassekonzentration, etc.) als Regelgrößen herangezogen werden oder aber die zu- oder abzuführenden Fluide werden nach einem vorgegebenen Profil oder als ein oder wiederholtem Puls zu- bzw. abgeführt.

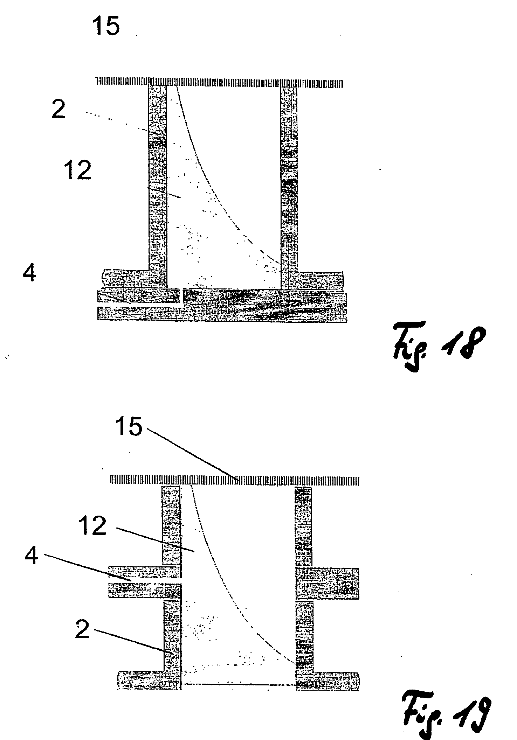

Die Figur 18 verdeutlicht noch mal die Primärausführung der Erfindung. Das zu dosierende Fluid wird durch eine Öffnung im Reaktorboden angeboten. Eine Ebene unter dem Reaktorboden beinhaltet die Fluidkanäle 4, durch die die Fluide zu den einzelnen Reaktoren 2 transportiert werden. Durch eine gasdurchlässige Membran 15 kann für eine sterile Gaszufuhr in die Reaktoren gesorgt werden. Die durch die Fluidkanäle angebotenen Tropfen werden von der durch das Schütteln erzeugten Flüssigkeitssichel 12 in die Flüssigkeit aufgenommen.

Bei in Figur 19 gezeigten Variante der Mikroreaktoren wird das zu dosierende Fluid durch eine Öffnung auf halber Reaktorhöhe angeboten. Eine Mittelebene beinhaltet die Fluidkanäle 4, durch die die Fluide zu den einzelnen Reaktoren 2 transportiert werden. Durch eine gasdurchlässige Membran 15 kann für eine sterile Gaszufuhr in die Reaktoren gesorgt werden. Die durch die Fluidkanäle angebotenen Tropfen werden von der durch das Schütteln erzeugten Flüssigkeitssichel 12 in die Flüssigkeit aufgenommen.

### Bezugszeichenliste:

- 1.: Mikroreaktor Array
- 2.: Mikroreaktor
- 3.: Reservoir für zu- oder abgeführtes Medium
- 4.: Fluidkanal / Schlauchverbindung
- 5.: Fluidebene
- 6.: Steuerungsebene
- 7.: Pneumatisch gesteuerter Kanal
- 8.: Flexible Membran
- 9.: beliebiger Aktuator
- 10.: Semipermeable Membran
- 11.: Fluidvorkammer
- 12.: Flüssigkeitssichel
- 13.: modifizierte Oberfläche
- 14.: Deckel des Mikroreaktors
- 15.: Belüftungseinlass des Reaktors
- 16.: Heizelemente
- 17.: Piezoelement
- 18.: Laser
- 19.: Fokussierter Laserstrahl
- 20.: Elektromagnetisch oder thermisch aktuiertes Material
- 21.: Dosierungsöffnung
- 22.: Dosiereinheit / Pipetierrobotor
- 23.: Justageöffnungen
- 24.: Optische Ausleseeinheit
- 25.: Optode (ändert Fluoreszenzeigenschaften in Abhängigkeit chemischer Größen z.B. pH-Wert)
- 26.: Regeleinheit / Computer

## Patentansprüche

1. Vorrichtung mit einem Mikroreaktoren-Array (1), der mehrere Behältnisse (3) aufweist, wobei jedes Behältnis des Arrays mindestens einen Kanal (4) aufweist, der jeweils eine Fluidleitung zum Behältnis bildet, wobei jeder Kanal ein Ventil aufweist, welches eine individuelle Zu- bzw. Abfuhr von Reaktionsfluiden in einzelne Reaktionskammern (2) des Mikroreaktoren-Arrays ermöglicht, und wobei der Arräy eine Pumpe aufweist, die dazu dient, die Reaktionsfluide in die Reaktionskammern zu befördern, ***dadurch gekennzeichnet, dass*** die Vorrichtung einen mechanischen Schüttler aufweist und die Zudosierung erfolgen kann, ohne den Schüttelvorgang zu unterbrechen und wobei die Reaktionskammern mit einer gasdurchlässigen Membran (10) oder einem gasdurchlässigen Deckel (14) abgedeckt sind.

2. Vorrichtung nach Anspruch 1, ***dadurch gekennzeichnet, dass*** die Behältnisse in einer Ebene angeordnet sind und der Kanal (4) in der Ebene der Behältnisse angeordnet ist

3. Vorrichtung nach Anspruch 1, ***dadurch gekennzeichnet, dass*** die Behältnisse in einer Ebene angeordnet sind und der Kanal (4) unter der Ebene der Behältnisse angeordnet ist.

4. Vorrichtung nach Anspruch 1, ***dadurch gekennzeichnet, dass*** der Übergang zwischen Kanal (4) und Behältnis oberhalb der Befüllebene des Behältnisses angeordnet ist.

5. Vorrichtung nach Anspruch 4, ***dadurch gekennzeichnet, dass*** der Array eine Abdeckung aufweist, in der der Kanal (4) angeordnet ist.

6. Vorrichtung nach einem der vorhergehenden Ansprüche, ***dadurch gekennzeichnet, dass*** ein Ventil oder eine Pumpe durch einen fokussierten Lichtstrahl (19) angesteuert sind.

7. Vorrichtung nach einem der vorhergehenden Ansprüche, ***dadurch gekennzeichnet, dass*** der Kanal eine durch Druck verformbare Wandung (8) als Ventil oder Pumpe aufweist.

8. Vorrichtung nach einem der vorhergehenden Ansprüche, ***dadurch gekennzeichnet, dass*** Vertiefungen auf dem Array (1) als pneumatische Anschlüsse ausgebildet sind.

9. Vorrichtung nach einem der vorhergehenden Ansprüche, ***dadurch gekennzeichnet, dass*** im Kanal oder im Behältnis eine teildurchlässige Membran (10) angeordnet ist.

10. Vorrichtung nach einem der vorhergehenden Ansprüche, ***dadurch gekennzeichnet, dass*** mindestens eine Fläche des Arrays (1) als optischer Zugang ausgebildet ist.

11. Vorrichtung nach einem der vorhergehenden Ansprüche, ***dadurch gekennzeichnet, dass*** der Array Sensoren (25) in den einzelnen Behältnissen zur Erfassung von Messdaten aufweist.

12. Vorrichtung nach einem der vorhergehenden Ansprüche, ***dadurch gekennzeichnet, dass*** der Schüttler mindestens eine in den Mikroreaktoren-Array (1) integrierte Pumpe antreibt.

13. Vorrichtung nach einem der vorhergehenden Ansprüche, ***dadurch gekennzeichnet, dass*** sie eine Mikrotiterplatte und eine Steuerungsebene (6) mit Ventilen aufweist, wobei die Steuerungsebene neben Ventilen zur Kontrolle des Durchflusses auch Pumpen für den eigentlichen Fluidtransport aufweist.

14. Vorrichtung nach einem der vorhergehenden Ansprüche, ***dadurch gekennzeichnet, dass*** eine Fluidebene (5) und eine Steuerungsebene (6) modular kombiniert sind.

15. Verfahren zur Verwendung einer Vorrichtung nach einem der vorhergehenden Ansprüche zur Zu- bzw. Abfuhr von Fluiden in Mikroreaktoren Arrays (1) durch mehrere Fluidkanäle, die in einzelne Mikroreaktoren (2) führen, ***dadurch gekennzeichnet, dass*** die Fluidkanäle individuell gesteuert und geregelt werden, wobei die Zudosierung erfolgt, ohne den Schüttelvorgang zu unterbrechen, und die Reaktionskammern jeweils teilweise mit Flüssigkeit und teilweise mit Gas gefüllt sind.

16. Verfahren nach Anspruch 15 zur Verwendung einer Vorrichtung nach Anspruch4, ***dadurch gekennzeichnet, dass*** eine Fluidmenge am Einlass des Kanals vorgehalten wird, der Einlass des Kanals bei unbewegter Flüssigkeit nicht mit der Flüssigkeit in Berührung steht und nur beim Bewegen der Reaktionsflüssigkeit die Sichel (12) der Flüssigkeit bis zum Einlass des Kanals reicht, so dass durch die Sichel der Flüssigkeitsoberfläche eine Fluidmenge abgenommen wird.

17. Verfahren nach einem der Ansprüche 15 oder 16, ***dadurch gekennzeichnet, dass*** die zuzuführenden Fluide in einer Vertiefung einer der Reaktionskammern (2), auf dem Mikroreaktoren Array (1) vorgehalten werden.

## Claims

1. A device with a microreactor array (1) which comprises a plurality of containers (3), wherein each container of the array comprises at least one channel (4) which respectively forms a fluid line to the container, wherein each channel comprises a valve which enables an individual supply and withdrawal of reaction fluids in respect of individual reaction chambers (2) of the microreactor array, and wherein the array comprises a pump which acts to transport the reaction fluids into the reaction chambers, ***characterized in that*** the device comprises a mechanical shaker and addition may be carried out without interrupting the shaking process and wherein the reaction chambers are covered with a gas-permeable membrane (10) or with a gas-permeable cover (14).

2. The device as claimed in claim 1, ***characterized in that*** the containers are disposed in a layer and the channel (4) is disposed in the layer for the containers.

3. The device as claimed in claim 1, ***characterized in that*** the containers are disposed in a layer and the channel (4) is disposed below the layer for the containers.

4. The device as claimed in claim 1, ***characterized in that*** the transition between the channel (4) and the container is disposed above the filling level of the container.

5. The device as claimed in claim 4, ***characterized in that*** the array comprises a cover in which the channel (4) is disposed.

6. The device as claimed in one of the preceding claims, ***characterized in that*** a valve or a pump is controlled by means of a focused beam of light (19).

7. The device as claimed in one of the preceding claims, ***characterized in that*** the channel comprises a wall (8) which can be deformed by pressure, as a valve or pump.

8. The device as claimed in one of the preceding claims, ***characterized in that*** indentations on the array (1) are configured as pneumatic connections.

9. The device as claimed in one of the preceding claims, ***characterized in that*** a semi-permeable membrane (10) is disposed in the channel or in the container.

10. The device as claimed in one of the preceding claims, ***characterized in that*** at least one surface of the array (1) is configured as an optical access.

11. The device as claimed in one of the preceding claims, ***characterized in that*** the array comprises sensors (25) in the individual containers for the acquisition of measurement data.

12. The device as claimed in one of the preceding claims, ***characterized in that*** the shaker drives at least one pump integrated into the microreactor array (1).

13. The device as claimed in one of the preceding claims, ***characterized in that*** it comprises a microreactor and a control layer (6) with valves, wherein in addition to valves to control the throughput, the control layer also comprises pumps for actual fluid transport.

14. The device as claimed in one of the preceding claims, ***characterized in that*** a fluid layer (5) and a control layer (6) are combined in a modular manner.

15. A method for using a device as claimed in one of the preceding claims for supplying or withdrawing fluids in microreactor arrays (1) through a plurality of fluid channels which lead into individual microreactors (2), ***characterized in that*** the fluid channels are individually controlled and regulated, whereupon addition is carried out without interrupting the shaking process, and the reaction chambers are respectively partially filled with liquid and partially filled with gas.

16. The method as claimed in claim 15 for using a device as claimed in claim 4, ***characterized in that*** a quantity of fluid is maintained at the inlet to the channel, the inlet to the channel does not come into contact with the liquid when the liquid is not in motion and the meniscus (12) of the liquid only extends to the inlet to the channel when the reaction liquid is in motion, so that a quantity of fluid is removed by means of the meniscus of the liquid surface.

17. The method as claimed in claim 15 or claim 16, ***characterized in that*** the fluids to be supplied are maintained in an indentation of one of the reaction chambers (2) on the microreactor array (1).

## Revendications

1. Dispositif comportant un réseau de microréacteurs (1) qui présente plusieurs réservoirs (3), chaque réservoir du réseau présentant au moins un canal (4) qui constitue respectivement une conduite de fluide vers le réservoir, chaque canal présentant une soupape qui permet un apport ou une évacuation individuel de fluides réactifs dans des chambres de réaction distinctes (2) du réseau de microréacteurs et le réseau présentant une pompe qui sert à transporter les fluides réactifs dans les chambres de réaction, ***caractérisé en ce que*** le dispositif présente un agitateur mécanique et que le dosage peut avoir lieu sans interrompre le processus d'agitation, les chambres de réaction étant recouvertes d'une membrane perméable au gaz (10) ou d'un couvercle (14) perméable au gaz.

2. Dispositif selon la revendication 1, ***caractérisé en ce que*** les réservoirs sont disposés dans un plan et le canal (4) est disposé dans le plan des réservoirs.

3. Dispositif selon la revendication 1, ***caractérisé en ce que*** les réservoirs sont disposés dans un plan et le canal (4) est disposé en-dessous du plan des réservoirs.

4. Dispositif selon la revendication 1, ***caractérisé en ce que*** la transition entre le canal (4) et le réservoir est disposée au-dessus du plan de remplissage du réservoir.

5. Dispositif selon la revendication 4, ***caractérisé en ce que*** le réseau présent un recouvrement dans lequel le canal (4) est disposé.

6. Dispositif selon une des revendications précédentes, ***caractérisé en ce* qu'**une soupape ou une pompe est commandée par un rayon lumineux focalisé (19).

7. Dispositif selon une des revendications précédentes, ***caractérisé en ce que*** le canal présente une paroi (8) déformable par pression faisant office de soupape ou de pompe.

8. Dispositif selon une des revendications précédentes, ***caractérisé en ce que*** des creux faisant office de raccordements pneumatiques sont pratiqués sur le réseau (1).

9. Dispositif selon une des revendications précédentes, *caractérisé en ce qu*'une membrane partiellement perméable (10) est disposée dans le canal ou dans le réservoir.

10. Dispositif selon une des revendications précédentes, *caractérisé en ce qu*'au moins une surface du réseau (1) se présente sous forme d'un accès optique.

11. Dispositif selon une des revendications précédentes, ***caractérisé en ce que*** le réseau présente des capteurs (25) dans les différents réservoirs pour détecter des données de mesure.

12. Dispositif selon une des revendications précédentes, ***caractérisé en ce que*** l'agitateur entraîne au moins une pompe intégrée dans le réseau de microréacteurs (1).

13. Dispositif selon une des revendications précédentes, *caractérisé en ce qu*'il présente une plaque de micro-titrage et un plan de commande (6) avec des soupapes, le plan de commande présentant, outre des soupapes de contrôle du débit, également des pompes pour le transport de fluide proprement dit.

14. Dispositif selon une des revendications précédentes, ***caractérisé en ce qu'***un plan de fluide (5) et un plan de commande (6) sont combinés de manière modulaire.

15. Procédé d'utilisation d'un dispositif selon une des revendications précédentes pour l'apport ou l'évacuation de fluides dans des réseaux de microréacteurs (1) par plusieurs canaux de fluide qui mènent dans différents microréacteurs (2), ***caractérisé en ce que*** les canaux de fluides sont commandés et régulés individuellement, le dosage ayant lieu sans interrompre le processus d'agitation et les chambres de réaction étant respectivement remplies partiellement de liquide et partiellement de gaz.

16. Procédé selon la revendication 15 pour l'utilisation d'un dispositif selon la revendication 4, ***caractérisé en ce* qu'**une quantité de fluide est retenue à l'entrée du canal, que l'entrée du canal, lorsque le liquide ne bouge pas, n'est pas en contact avec le liquide et que c'est seulement en cas de mouvement du liquide réactif que le croissant (12) de liquide va jusqu'à l'entrée du canal, de sorte qu'une quantité de fluide est prélevée par le croissant dans la surface du liquide.

17. Procédé selon une des revendications 15 ou 16, ***caractérisé en ce que*** les fluides à acheminer sont retenus dans un creux d'une des chambres de réaction (2) sur le réseau de microréacteurs (1).
